# EUROPÄISCHE PATENTSCHRIFT

(11) **EP 0 793 946 B1**
(45) Veröffentlichungstag und Bekanntmachung des Hinweises auf die Patenterteilung: **26.07.2000**
(21) Anmeldenummer: 97101271.1
(22) Anmeldetag: 28.01.1997
(51) Int. Cl.: A61B 17/28, A61B 18/14

(54) **Bipolares chirurgisches Fassinstrument**
Bipolar surgical forceps
Pince chirurgicale bipolaire

(30) Priorität: 06.03.1996 DE 19608716
(43) Veröffentlichungstag der Anmeldung: 10.09.1997
(73) Patentinhaber: Aesculap AG & Co. KG, 78532 Tuttlingen (DE)
(72) Erfinder: Mayenberger, Rupert, 78239 Rielasingen (DE)
(74) Vertreter: Böhme, Ulrich, Dr. Dipl.-Phys.

(56) Entgegenhaltungen:
- EP-A- 0 624 348
- WO-A-95/02366
- WO-A-95/05125
- DE-A- 4 312 284
- DE-U- 29 604 191

## Beschreibung

Die Erfindung betrifft ein bipolares chirurgisches Faßinstrument mit zwei verschwenkbar am Ende eines Rohrschafts gelagerten Armen, die durch Kniehebelelemente durch eine im Rohrschaft verschiebliche Schub- und Zugstange gegenläufig koaxial verschwenkbar sind, wobei die Schub- und Zugstange und die Kniehebelelemente beider Arme aus elektrisch leitendem Material bestehen, wobei der erste Arm elektrisch leitend mit dem ihm zugeordneten Kniehebelelement verbunden ist, wobei der zweite Arm über seine Schwenklagerung elektrisch leitend mit dem Rohrschaft verbunden ist und wobei der erste Arm gegenüber dem zweiten Arm und gegenüber dem Rohrschaft elektrisch isoliert an dem Rohrschaft drehbar gelagert ist.

Insbesondere bei endoskopischen Operationen ist es erwünscht, Rohrschaftinstrumente einzusetzen, bei denen gegeneinander verschwenkbare Arme eines Werkzeugs, beispielsweise Zangenschenkel oder Schenkel einer Schere, getrennt mit den verschiedenen Polen einer Hochfrequenzspannungsquelle verbunden werden können, so daß zwischen den elektrisch voneinander isolierten Armen dieses Werkzeugs Hochfrequenzströme zum Koagulieren und Schneiden von dazwischenliegendem Gewebe fließen können.

Es ist dabei äußerst schwierig, bei den kleinen Dimensionen derartiger Instrumente eine zuverlässige elektrische Isolierung der beiden Arme einerseits und einen einfachen Aufbau andererseits zu erreichen.

So ist es beispielsweise aus der US-Patentschrift 5,391,166 bekannt, in einem Rohrschaft eines Faßinstruments zwei Arme verschwenkbar zu lagern. Um diese elektrisch voneinander zu trennen, sind komplizierte Einzelteile notwendig, es werden insbesondere Isolierzwischenstücke, Isolierlagerstifte und isolierende Beschichtungen der Arme benötigt, wobei diese isolierenden Beschichtungen nur partiell vorgesehen sein dürfen. Bei der bekannten Konstruktion ist außerdem vorgesehen, daß zwei elektrische Zuleitungen im Inneren des Rohrschafts angeordnet werden müssen, diese müssen mit einer Steckverbindung mit einem speziellen Adapter verbunden werden. Insgesamt ergibt sich dadurch eine recht komplizierte Konstruktion, die es auch nicht ermöglicht, in der herkömmlichen Weise mit einer einzigen Schub- und Zugstange auszukommen, da zwei getrennte elektrische Zuführungen im Inneren des Rohrschafts notwendig sind.

Dies gilt auch bei einer anderen Konstruktion, die in der US-Patentschrift 5,352,222 beschrieben ist. Auch hier sind zwei getrennte Zuleitungsstäbe notwendig, außerdem müssen beide Arme des Werkzeugs in komplizierter Weise sandwichartig aufgebaut sein, da die einander gegenüberliegenden Klingen des scherenartigen Instruments gegenüber den eigentlichen Armen durch eine isolierende Zwischenschicht zu trennen sind. Auch hier ergibt sich eine sehr komplizierte Herstellung, wobei bei den kleinen Dimensionen derartiger Instrumente auch Fehlfunktionen nicht auszuschließen sind.

Ein gattungsgemäßes bipolares chirurgisches Faßinstrument ist aus der WO-A-9505125 bekannt. Die Arme des Faßinstrumentes bestehen dabei im wesentlichen aus Kunststoff mit eingelegten Metallteilen.

Ausgehend von diesem Stand der Technik liegt der Erfindung die Aufgabe zugrunde, ein gattungsgemäßes Faßinstrument mit mechanisch unkompliziertem Aufbau zu schaffen, welches insbesondere eine hohe mechanische Festigkeit aufweist.

Diese Aufgabe wird bei einem bipolaren chirurgischen Faßinstrument der eingangs beschriebenen Art erfindungsgemäß dadurch gelöst, daß die Schub- und Zugstange und die Kniehebelelemente beider Arme elektrisch leitend miteinander verbunden sind und daß der zweite Arm über ein eingesetztes Isolierteil mit dem ihm zugeordneten Kniehebelelement elektrisch isoliert verbunden ist.

Bei dieser Konstruktion werden also beide Arme in an sich bekannter Weise über Kniehebelelemente gegenläufig verschwenkt, wobei diese Kniehebelelemente gemeinsam über eine einzige Schub- und Zugstange angetrieben werden. Diese Schub- und Zugstange bildet den einen elektrischen Pol der Hochfrequenzspannungsquelle, der andere Pol wird durch den Rohrschaft selbst gebildet.

Die Kniehebelelemente des Antriebs für beide Arme sind gemeinsam mit der einen Spannungsquelle verbunden, lediglich einer der beiden Arme ist gegenüber diesem Kniehebelelement durch Zwischenschaltung des Isolierteils elektrisch isoliert. Dieser zweite Arm ist seinerseits mit dem Rohrschaft elektrisch leitend verbunden. Um hier einen Kurzschluß zu vermeiden, genügt es, wenn der andere Arm, also der elektrisch mit der Schub- und Zugstange in Verbindung stehende erste Arm gegenüber der Schwenklagerung elektrisch isoliert ist und gegenüber dem anderen Arm.

Eine solche Konstruktion läßt sich mit einfachen Mitteln realisieren, die mechanischen Antriebsbewegungen bleiben im wesentlichen gleich wie bei herkömmlichen Rohrschaftinstrumenten, die nicht als bipolare Instrumente ausgebildet sind.

Besonders vorteilhaft ist es, wenn gemäß einer bevorzugten Ausführungsform beide Arme über eine leitende Lagerwelle am Rohrschaft drehbar gelagert sind und wenn der erste Arm gegenüber dieser Lagerwelle elektrisch isoliert ist. Diese elektrisch leitende Lagerwelle kann nämlich dann die elektrische Verbindung zwischen dem zweiten Arm und dem Rohrschaft herstellen, wobei der erste Arm jedoch weiterhin gegenüber dem Rohrschaft elektrisch isoliert ist.

Vorzugsweise wird zwischen der Lagerwelle und dem ersten Arm eine die Lagerwelle umgebende Isolierhülse angeordnet, um diese Isolierung des ersten Arms gegenüber der Lagerwelle zu erreichen.

Besonders vorteilhaft ist es dabei, wenn die Isolierhülse Teil des Isolierteils ist, es ist nämlich dann nicht notwendig, hier ein zusätzliches Teil zur Isolierung des ersten Arms gegenüber der Lagerwelle und damit gegenüber dem Rohrschaft vorzusehen.

Vorzugsweise wird das Isolierteil aus Keramik hergestellt, es wäre aber auch möglich, das Isolierteil aus Kunststoff herzustellen.

Bei einer bevorzugten Ausführungsform ist vorgesehen, daß das Isolierteil plattenförmig ausgebildet ist und im Schwenklagebereich zwischen dem zweiten Arm und dem zugeordneten Kniehebelelement in Längsrichtung sandwichartig eingebettet ist. Man erhält also in Richtung der Schwenkachse gesehen einen dreilagigen Aufbau Kniehebelelement/Isolierteil/zweiter Arm.

Dabei ist es vorteilhaft, wenn das Isolierteil an dem Kniehebelelement einerseits und an dem zweiten Arm andererseits über Stirnflächen flächig anliegt. Über diese Stirnfläche können Drehmomente von dem Kniehebelelement auf den zweiten Arm übertragen werden.

Weiterhin ist es günstig, wenn die sandwichförmig aneinanderliegenden Bereichen des zweiten Arms, des Isolierteils und des dem zweiten Arm zugeordneten Kniehebelelements miteinander fluchtende Öffnungen für eine durchgehende Lagerwelle aufweisen. Beim Einsetzen der Lagerwelle werden dann diese drei Teile durch die flächig aneinanderliegenden Stirnflächen drehfest miteinander verbunden und können Drehmomente vom Kniehebelelement auf den zweiten Arm übertragen, ohne daß zusätzliche Fixierungen notwendig sind.

Es kann dabei weiterhin vorgesehen sein, daß bei einer leitend mit dem Rohrschaft verbundenen Lagerwelle das Isolierteil die benachbarten Bereiche des dem zweiten Arm zugeordneten Kniehebels gegenüber der Lagerwelle elektrisch isolierend umgibt. Separate Teile zur Isolation dieses Kniehebelelements gegenüber der Lagerwelle sind dann nicht notwendig.

Besonders vorteilhaft ist es auch, wenn der erste Arm und das ihm zugeordnete Kniehebelelement einstückig ausgebildet sind.

Das Isolierteil kann so geformt sein, daß der zweite Arm, das Isolierteil und das zugeordnete Kniehebelelement nach dem Einsetzen der Lagerwelle ein starres Bauteil bilden, das in seiner Form spiegelbildlich zum ersten Arm und dem ihm zugeordneten Kniehebelelement ausgebildet ist. Man erhält damit zwei Arme, die in ihren Abmessungen herkömmlichen Armen entsprechen, wobei aber der eine Arm durch das Einsetzen des Isolierteils unterbrochen wird, so daß gegenüber der Lagerwelle, dem Kniehebelelement und dem anderen Arm eine elektrische Isolierung möglich wird.

Zusätzlich kann bei einer bevorzugten Ausführungsform vorgesehen sein, daß in den Rohrschaft eine Isolierhülse eingesteckt ist, die die Schub- und Zugstange gegenüber dem Rohrschaft isoliert und die zwischen parallel nach vorn gerichteten Schenkeln das Ende der Schub- und Zugstange, die Kniehebelelemente sowie die Schwenklagerung des ersten und des zweiten Arms aufnimmt. Diese Isolierhülse zentriert zusätzlich die Schub- und Zugstange im Rohrschaft und gewährleistet eine elektrische Isolierung auch des Kniehebelantriebs gegenüber dem Rohrschaft.

Die nachfolgende Beschreibung bevorzugter Ausführungsformen dient im Zusammenhang mit der Zeichnung der näheren Erläuterung. Es zeigen:

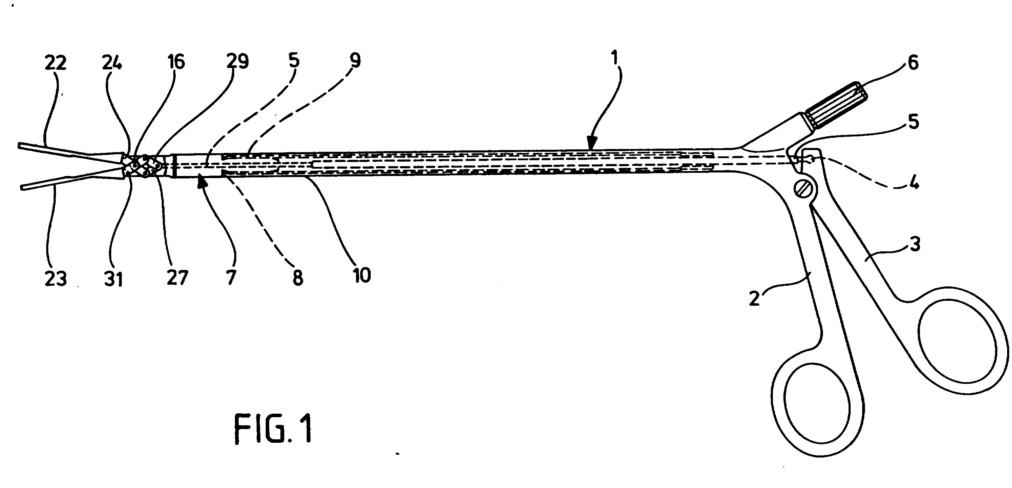

- Figur 1:: Eine seitliche Gesamtansicht eines bipolaren chirurgischen Faßinstruments;

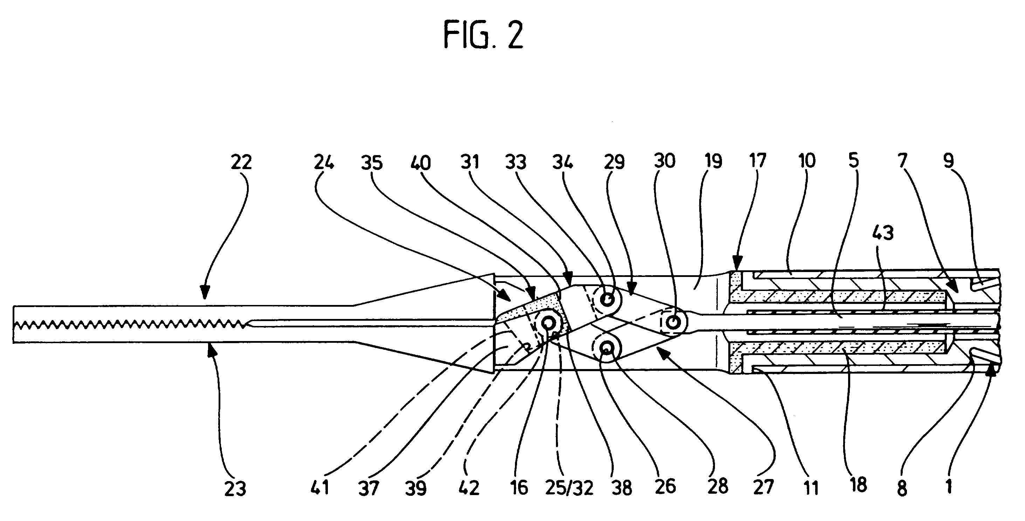

- Figur 2:: eine vergrößerte Seitenansicht des vorderen Bereichs des Instruments der Figur 1 in einer Teillängsschnittdarstellung;

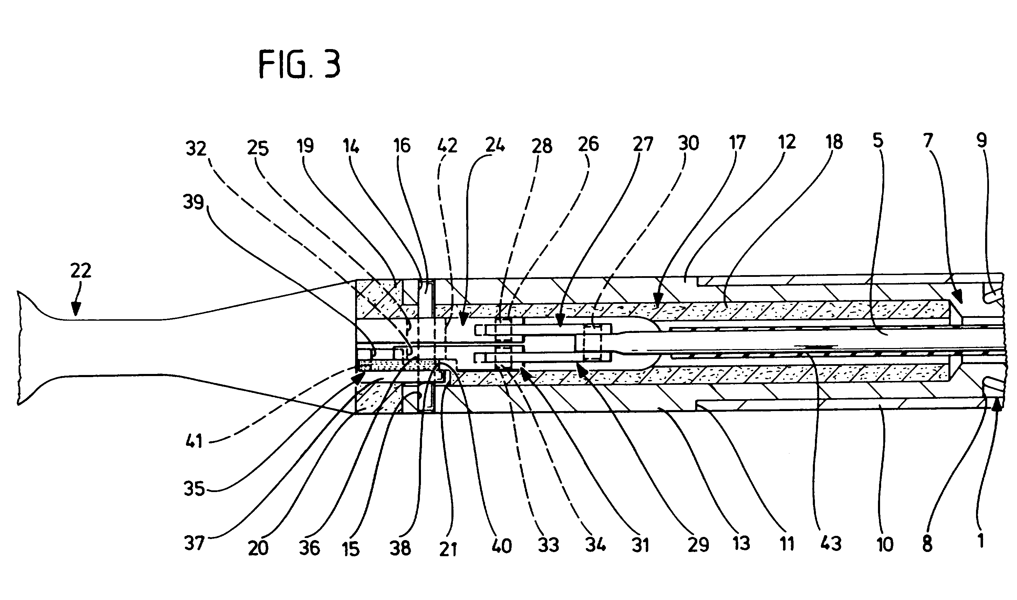

- Figur 3:: eine Ansicht ähnlich Figur 2 bei um 90° um die Längsachse gedrehtem Instrument und
- Figur 4:: eine Explosionsansicht des vorderen Teils des Instruments der Figur 1.

Das in der Zeichnung dargestellte Rohrschaftinstrument umfaßt ein längliches Rohr 1, welches an seinem rückwärtigen Ende fest mit einer Griffbranche 2 verbunden ist. An dieser ist um eine quer zur Längsrichtung des Rohrs 1 verlaufende Drehachse eine zweite Griffbranche 3 verschwenkbar gelagert, die über eine Kugelkopfverbindung 4 mit einer im Inneren des Rohrs 1 verlaufenden Schub- und Zugstange 5 verbunden ist.

Das Rohr 1 ist über einen Steckanschluß 6 mit dem ersten Pol einer in der Zeichnung nicht dargestellten Hochfrequenzspannungsquelle verbindbar, ebenso kann die Schub- und Zugstange 5 über eine in der Zeichnung nicht erkennbare Verbindung mit dem anderen Pol dieser Hochfrequenzspannungsquelle verbunden werden.

Das Rohr 1 und die Schub- und Zugstange 5 sind elektrisch voneinander isoliert, dies kann im Bereich der Griffbranchen 2 und 3 beispielsweise dadurch erfolgen, daß die Schub- und Zugstange 5 in ihrer Länge durch ein isolierendes Zwischenteil unterbrochen ist und daß die Verbindung der Schub- und Zugstange 5 mit der Spannungsquelle vor dieser Unterbrechungsstelle erfolgt.

In das vordere Ende des Rohrs 1 ist ein Rohrstück 7 aus einem elektrisch leitenden Material eingeschoben, vorzugsweise ebenfalls aus Metall bestehend. Dieses Rohrstück 7 weist an seinem in das Rohr 1 eingeschobene Ende eine Ringnut 8 auf, in die durch Längsschnitte zungenförmig ausgebildete Wandteile 9 des Rohrs 1 derart eingreifen, daß ein Herausziehen des Rohrstücks 7 aus dem Rohr 1 nur möglich wird, wenn diese zungenförmigen Wandteile 9 elastisch nach außen gebogen werden. Ein solches Verbiegen der Wandteile 9 läßt sich durch eine die Wandteile 9 im Bereich der Ringnut 8 überfangende Hülse 10 verhindern, die auf dem Rohr 1 längsverschieblich gelagert ist und normalerweise gegen eine Stufe 11 des Rohrstücks 7 stößt. Wird diese Hülse 10 jedoch so weit zurückgezogen, daß Wandteile 9 und Ringnut 8 freigegeben werden, können die Wandteile 9 elastisch nach außen gebogen werden, so daß dann das Rohrstück 7 aus dem Rohr 1 herausgezogen werden kann.

Das Rohrstück 7 trägt an seinem aus dem Rohr 1 hervorstehenden Ende zwei parallel nach vorne gerichtete Schenkel 12, 13, an deren freien Enden sich zwei miteinander ausgerichtete Öffnungen 14, 15 zur Aufnahme einer die beiden Schenkel 12, 13 verbindenden Lagerwelle 16 aus elektrisch leitendem Material angeordnet sind.

In das Rohrstück 7 ist ein Isolierkörper 17 aus Spritzkeramik eingeschoben, der ähnlich geformt ist wie das Rohrstück 7 selbst. Ein hülsenförmiger Teil 18 taucht in den geschlossenen Teil des Rohrstücks 7 ein, zwei einstückig mit dem hülsenförmigen Teil 18 verbundene parallele Schenkel 19, 20, liegen jeweils an der Innenseite der Schenkel 12, 13 des Rohrstücks 12 an. Dabei überragen die Schenkel 19 und 20 die Schenkel 12 und 13 geringfügig. Im Bereich der Öffnungen 14 und 15 in den Schenkeln 12 beziehungsweise 13 weisen auch die Schenkel 19 und 20 Ausnehmungen 21 auf, durch die die Lagerwelle 16 hindurchtreten kann.

Im Inneren des hülsenförmigen Teils 18 des Isolierkörpers 17 verläuft die Schub- und Zugstange 5, die zwischen den beiden Schenkeln 19 und 20 endet. Diese Schub- und Zugstange wird durch den Isolierkörper 17 zentriert und gegenüber dem Rohrstück 7 und damit dem Rohr 1 elektrisch isoliert. Zusätzlich ist die Schub- und Zugstange 5 von einem Isolierschlauch 43 umgeben.

Die Lagerwelle 16 bildet eine Schwenklagerung für zwei Arme 22 und 23 eines zangenförmigen oder gegebenenfalls auch scherenförmigen Instruments, die durch die Lagerwelle 16 gegenläufig verschwenkbar am Rohr 1 gelagert sind. Der erste Arm 22 besteht aus einem elektrisch leitenden Material, insbesondere aus Metall, und ist einstückig mit einer Verlängerung verbunden, die ein Kniehebelelement 24 ausbildet. In dieser Verlängerung befindet sich eine Öffnung 25, durch die die Lagerwelle 16 hindurchtritt, und eine weitere Öffnung 26, durch die ein das Kniehebelelement 24 mit einem weiteren Kniehebelelement 27 gelenkig verbindender Lagerstift 28 gesteckt werden kann, der seinerseits mit seinem gegenüberliegenden Ende gelenkig mit dem Ende der Schub- und Zugstange 5 verbunden ist.

Das Kniehebelelement 24 und das Kniehebelelement 27 bilden gemeinsam einen Kniehebel, dessen Winkel durch Vor- und Zurückschieben der Schub- und Zugstange 5 verändert werden kann, da das Kniehebelelement 24 im Bereich der Öffnung 25 gegenüber dem Rohr 1 ortsfest verschwenkbar gelagert ist. Diese Verschwenkung des Kniehebelelements 24 führt zu einer Verschwenkung des ersten Arms 22, da das Kniehebelelement 24 einstückig mit dem ersten Arm 22 ausgebildet ist.

Ein ähnlicher Kniehebel wird ausgebildet durch ein dem Kniehebelelement 27 entsprechendes und mit der Schub- und Zugstange 5 über einen Lagerstift 30 gelenkig verbundenes Kniehebelelement 29, welches über einen Lagerstift 34 gelenkig mit einem weiteren Kniehebelelement 31 verbunden ist. Auch dieses Kniehebelelement entspricht dem Kniehebelelement 24 des ersten Arms, wie dieses weist es eine Öffnung 32 für den Durchtritt der Lagerwelle 16 und eine Öffnung 33 für die Aufnahme des Lagerstifts 34 auf, der die beiden Kniehebelelemente 29 und 31 gelenkig miteinander verbindet.

Im Unterschied zum Kniehebelelement 24 ist jedoch dieses Kniehebelelement nicht einstückig mit dem zweiten Arm 23 verbunden, sondern stellt ein separates Bauteil dar. Eine Verbindung zwischen diesem Kniehebelelement 31 und dem zweiten Arm 23 wird in diesem Falle durch ein zwischen die beiden Teile eingesetztes Isolierteil 35 hergestellt, das vorzugsweise aus Spritzkeramik besteht, jedoch auch aus Kunststoff hergestellt werden kann. Dieses Isolierteil 35 ist plattenförmig ausgebildet und liegt in Längsrichtung zwischen ebenfalls plattenförmigen Lappen 36 beziehungsweise 37 des Kniehebelelements 31 und des zweiten Arms 23. Es ergibt sich dadurch eine sandwichartige Struktur, bei der in Richtung der Lagerwelle 16 der Lappen 36, das Isolierteil 35 und der Lappen 37 flächig aneinander liegen.

Der Lappen 36 und der Lappen 37 weisen jeweils eine Stufe 38 beziehungsweise 39 auf, an denen die rückwärtige Stirnfläche 40 beziehungsweise die vordere Stirnfläche 41 des plattenförmigen Isolierteils 35 flächig anliegen.

An einer Seite ist an das plattenförmige Isolierteil 35 eine Hülse 42 angeformt, die die Öffnung 32 des Kniehebelelements 31 und die Öffnung 25 des Kniehebelelements 24 durchsetzt und die die Lagerwelle 16 umgibt, die dadurch gegenüber den beiden Kniehebelelementen 24 und 31 elektrisch isoliert ist.

Bis auf die Hülse 42 ist der erste Arm 22 mit dem angeformten Kniehebelelement 24 spiegelbildlich gleich geformt, wie das Bauteil, das aus dem zweiten Arm 23, dem Isolierteil 35 und dem Kniehebelelement 31 zusammengesetzt ist.

Die Kniehebelelemente 24, 27, 29 und 31 bestehen aus elektrisch leitendem Material und sind untereinander und mit der Schub- und Zugstange 5 elektrisch leitend verbunden. Dadurch ergibt sich eine elektrisch leitende Verbindung des ersten Arms 22 mit der Schub- und Zugstange 5. Andererseits ist der zweite Arm 23 durch das Isolierteil 35 gegenüber dem Kniehebelelement 31 und damit auch gegenüber der Schub- und Zugstange 5 elektrisch isoliert.

Die Lagerwelle 16 steht in elektrisch leitender Verbindung mit dem Rohrstück 7 und damit dem Rohr 1 und ausserdem mit dem Arm 23, sie wird jedoch durch das Isolierstück 35 und insbesondere dessen Hülse 42 gegenüber allen Kniehebelelementen 24, 27, 29 und 31 elektrisch isoliert, also auch gegenüber der Schub- und Zugstange 5. Damit ist eine elektrische Trennung der Arme 22 und 23 erreicht, und zwar ohne aufwendige Baumaßnahmen lediglich durch Einsetzen des Isolierteils 35. Durch diese Konstruktion ist es möglich, derartige Instrumente wahlweise als bipolare Instrumente oder ohne elektrische Anwendung einzusetzen.

Wenn der Arm 23 entsprechend der Ausgestaltung des Arms 22 einstückig mit dem Kniehebelelement 31 ausgebildet wird, fehlt die elektrische Isolation, so daß dieses Instrument dann in herkömmlicher Weise verwendet werden kann. Tauscht man diesen einstückigen Arm 23 gegen einen dreiteiligen Arm mit eingesetztem Isolierteil 35 aus, kann man bei sonst unveränderter Konstruktion die beiden Arme elektrisch voneinander isolieren und den einen Arm mit der Schub- und Zugstange 5 elektrisch verbinden, den anderen Arm mit dem Rohrstück 7 und damit dem Rohr 1.

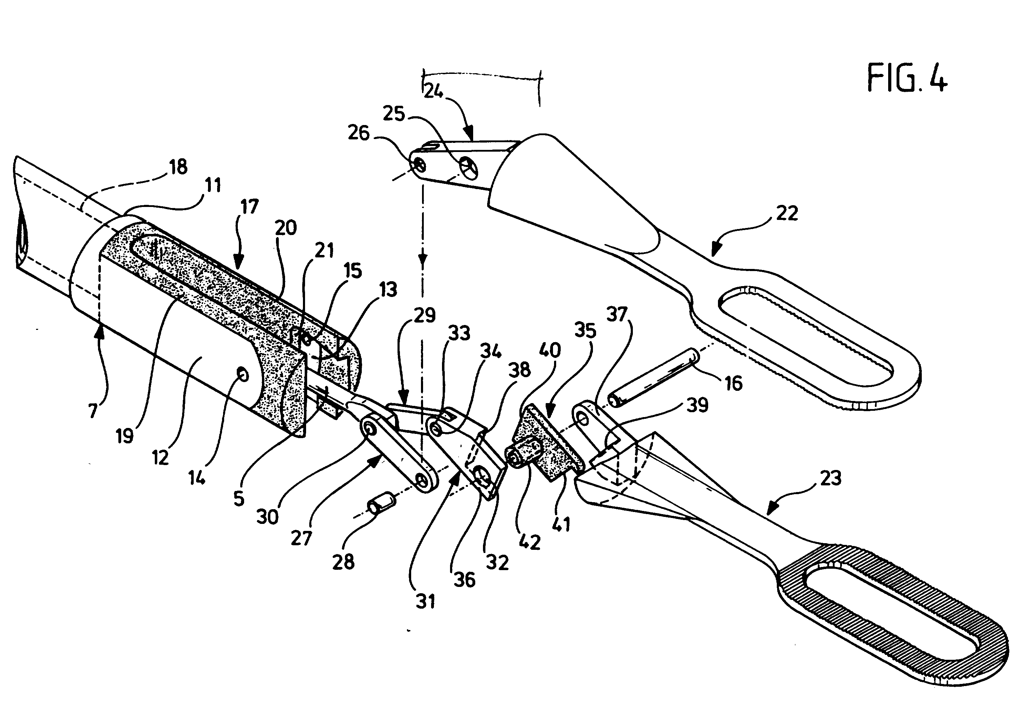

Durch diesen besonders einfachen Aufbau kann das Instrument auch in einfachster Weise zerlegt und gereinigt werden, dies wird insbesondere aus der Darstellung der Figur 4 deutlich, die zeigt, daß durch Herausziehen der Lagerwelle 16 und des Lagerstifts 28 eine weitgehende Zerlegung möglich ist, sobald die Schub- und Zugstange 5 nach vorne aus dem Rohrstück 7 herausgezogen wird.

## Patentansprüche

1. Bipolares chirurgisches Faßinstrument mit zwei verschwenkbar am Ende eines Rohrschafts gelagerten Armen (22, 23), die durch Kniehebelelemente (24, 27; 31, 29) durch eine im Rohrschaft verschiebliche Schub- und Zugstange (5) gegenläufig koaxial verschwenkbar sind,
- wobei die Schub- und Zugstange und die Kniehebelelemente beider Arme aus elektrisch leitendem Material bestehen;
- wobei der erste Arm (22) elektrisch leitend mit dem ihm zugeordneten Kniehebelelement (24) verbunden ist;
- wobei der zweite Arm (23) über seine Schwenklagerung (16) elektrisch leitend mit dem Rohrschaft (7, 1) verbunden ist;
- und wobei der erste Arm (22) gegenüber dem zweiten Arm (23) und gegenüber dem Rohrschaft (7, 1) elektrisch isoliert an dem Rohrschaft drehbar gelagert ist,
dadurch gekennzeichnet,
- daß die Schub- und Zugstange (5) und die Kniehebelelemente (24, 27; 31, 29) beider Arme (22, 23) elektrisch leitend miteinander verbunden sind und
- daß der zweite Arm (23) über ein eingesetztes Isolierteil (35) mit dem ihm zugeordneten Kniehebelelement (31) elektrisch isoliert verbunden ist.

2. Instrument nach Anspruch 1, dadurch gekennzeichnet, daß beide Arme (22, 23) über eine leitende Lagerwelle (16) am Rohrschaft (7, 1) drehbar gelagert sind und daß der erste Arm (22) gegenüber dieser Lagerwelle (16) elektrisch isoliert ist.

3. Instrument nach Anspruch 2, dadurch gekennzeichnet, daß zwischen der Lagerwelle (16) und dem ersten Arm (22) eine die Lagerwelle (16) umgebende Isolierhülse (42) angeordnet ist.

4. Instrument nach Anspruch 3, dadurch gekennzeichnet, daß die Isolierhülse (42) Teil des Isolierteils (35) ist.

5. Instrument nach einem der voranstehenden Ansprüche, dadurch gekennzeichnet, daß das Isolierteil (35) aus Keramik besteht.

6. Instrument nach einem der Ansprüche 1 bis 4, dadurch gekennzeichnet, daß das Isolierteil (35) aus Kunststoff besteht.

7. Instrument nach einem der voranstehenden Ansprüche, dadurch gekennzeichnet, daß das Isolierteil (35) plattenförmig ausgebildet ist und im Schwenklagerbereich zwischen dem zweiten Arm (23) und dem zugeordneten Kniehebelelement sandwichartig eingebettet ist.

8. Instrument nach Anspruch 7, dadurch gekennzeichnet daß das Isolierteil (35) an dem Kniehebelelement (31) einerseits und an dem zweiten Arm (23) andererseits über Stirnflächen (40, 41) flächig anliegt.

9. Instrument nach Anspruch 8, dadurch gekennzeichnet, daß die sandwichförmig aneinanderliegenden Bereiche des zweiten Arms (23), des Isolierteils (35) und des dem zweiten Arm (23) zugeordneten Kniehebelelements (31) miteinander fluchtende Öffnungen für eine durchgehende Lagerwelle (16) aufweisen.

10. Instrument nach Anspruch 9, dadurch gekennzeichnet, daß bei einer leitend mit dem Rohrschaft (7, 1) verbundenen Lagerwelle (16) das Isolierteil (35) die benachbarten Bereiche des dem zweiten Arm (23) zugeordneten Kniehebelelements (31) gegenüber der Lagerwelle (16) elektrisch isolierend umgibt.

11. Instrument nach einem der voranstehenden Ansprüche, dadurch gekennzeichnet, daß der erste Arm (22) und das ihm zugeordnete Kniehebelelement (24) einstückig ausgebildet sind.

12. Instrument nach einem der voranstehenden Ansprüche, dadurch gekennzeichnet, daß in den Rohrschaft (7, 1) eine Isolierhülse (17) eingesteckt ist, die die Schub- und Zugstange (5) gegenüber dem Rohrschaft (7, 1) isoliert und die zwischen parallel nach vorn gerichteten Schenkeln (19, 20) das Ende der Schub- und Zugstange (5), die Kniehebelelemente (24, 27, 29, 31) sowie die Schwenklagerung (16) des ersten und zweiten Arms (22 beziehungsweise 23) aufnimmt.

## Claims

1. A bipolar surgical gripping instrument having two arms (22, 23) which are pivotably mounted on the end of a tubular shank and which, by toggle-joint elements (24, 27; 31, 29), are coaxially pivotable in opposite directions by a push and pull rod (5) movable in the tubular shank,
- the push and pull rod and the toggle-joint elements of both arms being composed of electrically conductive material;
- the first arm (22) being connected to its associated toggle-joint element (24) in an electrically conductive manner;
- the second arm (23) being connected, via its pivot mounting (16), to the tubular shank (7, 1) in an electrically conductive manner;
- and the first arm (22) being pivoted on the tubular shank (7, 1) in a manner so as to be electrically insulated with respect to the second arm (23) and with respect to the tubular shank (7, 1),
characterised,
- in that the push and pull rod (5) is connected to the toggle-joint elements (24, 27; 31, 29) of both arms (22, 23) in an electrically conductive manner and
- in that the second arm (23) is connected, via an inserted insulating part (35), to its associated toggle-joint element (31) in an electrically insulated manner.

2. An instrument in accordance with Claim 1, characterised in that both arms (22, 23) are pivoted on the tubular shank (7, 1) via a conductive bearing spindle (16), and in that the first arm (22) is electrically insulated with respect to this bearing spindle (16).

3. An instrument in accordance with Claim 2, characterised in that an insulating sleeve (42) which surrounds the bearing spindle (16) is arranged between the bearing spindle (16) and the first arm (22).

4. An instrument in accordance with Claim 3, characterised in that the insulating sleeve (42) is part of the insulating part (35).

5. An instrument in accordance with any one of the preceding Claims, characterised in that the insulating part (35) is composed of ceramic.

6. An instrument in accordance with any one of Claims 1 to 4, characterised in that the insulating part (35) is composed of plastics material.

7. An instrument in accordance with any one of the preceding Claims, characterised in that the insulating part (35) is plate-shaped and in the pivot bearing region is inserted sandwich-like between the second arm (23) and its associated toggle-joint element.

8. An instrument in accordance with Claim 7, characterised in that end faces (40, 41) of the insulating part (35) contact, in a surface-to-surface manner, with the toggle-joint element (31) on the one hand and with the second arm (23) on the other hand.

9. An instrument in accordance with Claim 8, characterised in that the regions, lying against one another in the manner of a sandwich, of the second arm (23), of the insulating part (35) and of the toggle-joint element (31) associated with the second arm (23) have aligned openings for a through-passing bearing spindle (16).

10. An instrument in accordance with Claim 9, characterised in that in the case of a bearing spindle (16) conductively connected to the tubular shank (7, 1), the insulating part (35) encloses, in an electrically insulating manner, the neighbouring regions of the toggle-joint element (31), associated with the second arm (23), with respect to the bearing spindle (16).

11. An instrument in accordance with any one of the preceding Claims, characterised in that the first arm (22) is integral with its associated toggle-joint element (24).

12. An instrument in accordance with any one of the preceding Claims, characterised in that an insulating sleeve (17) is put into the tubular shank (7, 1), this insulating sleeve (17) insulating the push and pull rod (5) with respect to the tubular shank (7, 1) and receiving, between legs (19, 20) which are directed forwards in a parallel manner, the end of the push and pull rod (5), the toggle-joint elements (24, 27, 29, 31) and the pivot mounting (16) of the first and second arms (22 and 23 respectively).

## Revendications

1. Pince chirurgicale bipolaire, comprenant deux bras (22, 23) montés en pivotement à l'extrémité d'un fût tubulaire, lesdits bras étant susceptibles de pivoter coaxialement en sens opposés au moyen d'éléments de levier à genouillère (24, 27 ; 31, 29) à l'aide d'une tige poussée/tirée (5) en déplacement dans le fût tubulaire, dans lequel :
- la tige poussée/tirée et les éléments de levier à genouillère des deux bras sont réalisés en un matériau conducteur de l'électricité ;
- le premier bras (22) est relié de façon à conduire l'électricité à l'élément de levier à genouillère (24) qui lui est associé ;
- le deuxième bras (23) est relié de façon à conduire l'électricité au fût tubulaire (7, 1) via sa monture de pivotement (16) ; et
- le premier bras (22) est monté en rotation sur le fût tubulaire en étant électriquement isolé vis-à-vis du deuxième bras (23) et vis-à-vis du fût tubulaire ;
caractérisée en ce que :
- la tige poussée/tirée (5) et les éléments de levier à genouillère (24, 27; 31, 29) des deux bras sont reliés de façon à conduire l'électricité les uns aux autres, et
- le deuxième bras (23) est relié de façon à conduire l'électricité à l'élément de levier à genouillère qui lui est associé (31) par l'intermédiaire d'une pièce isolante (35) intégrée.

2. Pince selon la revendication 1, caractérisée en ce que les deux bras (22, 23) sont montés en rotation via un arbre de montage conducteur (16) sur le fût tubulaire (7, 1), et en ce que le premier bras (22) est isolé vis-à-vis de cet arbre de montage (16).

3. Pince selon la revendication 2, caractérisée en ce qu'une douille isolante (42) qui entoure l'arbre de montage (16) est agencée entre l'arbre de montage (16) et le premier bras (22).

4. Pince selon la revendication 3, caractérisée en ce que la douille isolante (42) fait partie de la partie isolante (35).

5. Pince selon l'une des revendications précédentes, caractérisée en ce que la partie isolante (35) est réalisée en céramique.

6. Pince selon l'une des revendications 1 à 4, caractérisée en ce que la partie isolante (35) est réalisée en matière plastique.

7. Pince selon l'une des revendications précédentes, caractérisée en ce que la partie isolante (35) est réalisée sous forme de plaque, et en ce qu'elle est noyée dans la région de la monture pivotante à la manière d'un sandwich entre le deuxième bras (23) et l'élément de levier à genouillère associé.

8. Pince selon la revendication 7, caractérisée en ce que la partie isolante (35) est appliquée à plat d'une part contre l'élément de levier à genouillère (31), et d'autre part contre le deuxième bras (23) via des faces frontales (40, 41).

9. Pince selon la revendication 8, caractérisée en ce que les régions reposant en forme de sandwich les unes sur les autres du deuxième bras (23), de la partie isolante (35), et de l'élément de levier à genouillère (31) associé au deuxième bras (23) présentent des ouvertures mutuellement alignées pour un arbre de montage traversant (16).

10. Pince selon la revendication 9, caractérisée en ce que dans le cas d'un arbre de montage (16) relié de manière à conduire l'électricité au fût tubulaire (7, 1), la partie isolante (35) entoure de façon électriquement isolante les zones voisines de l'élément de levier à genouillère (31) associé au deuxième bras (23) vis-à-vis de l'arbre de montage (16).

11. Pince selon l'une des revendications précédentes, caractérisée en ce que le premier bras (22) et l'élément de levier à genouillère (24) qui lui est associé sont réalisés d'une seule pièce.

12. Pince selon l'une des revendications précédentes, caractérisée en ce qu'il est prévu une douille isolante (17) enfichée dans le fût tubulaire (7, 1), qui isole la tige poussée/tirée (5) vis-à-vis du fût tubulaire (7, 1), et qui reçoit, entre des bras (19, 20) dirigés parallèlement vers l'avant, l'extrémité de la tige poussée/tirée (5), les éléments de levier à genouillère (24, 27, 29, 31), ainsi que la monture pivotante (16) du premier et du deuxième bras (22, respectivement 23).
